# EUROPEAN PATENT SPECIFICATION

(11) **EP 3 838 227 B1**
(45) Date of publication and mention of the grant of the patent: **25.02.2026**
(21) Application number: 19216465.5
(22) Date of filing: 16.12.2019
(51) Int. Cl.: A61F 2/36, A61B 17/72, A61F 2/40, A61F 2/28, A61F 2/38, A61F 2/30, A61F 2/46

(54) **CEMENT RETAINING IMPLANT STEM FOR PERMANENT FIXATION**
ZEMENTRÜCKHALTEIMPLANTATSCHAFT ZUR DAUERHAFTEN FIXIERUNG
TIGE D'IMPLANT DE RETENUE DE CIMENT POUR FIXATION PERMANENTE

(43) Date of publication of application: 23.06.2021
(73) Proprietor: Waldemar Link GmbH & Co. KG, 22339 Hamburg (DE)
(72) Inventor: LINK, Helmut D., 22397 Hamburg (DE); CAPANNA, Rodolfo, 50139 Firenze (IT)
(74) Representative: Alatis

(56) References cited:
- WO-A1-2019/171139
- DE-A1- 3 903 438
- FR-A1- 2 855 396
- US-A1- 2006 149 246
- US-A1- 2016 235 955

## Description

### TECHNICAL FIELD

The present disclosure relates to an implant stem comprising a recess interrupted by a barrier.

### BACKGROUND OF THE INVENTION

One important objective in orthopedic surgery is a reliable anchorage of an implant within a bone. For implantation into long bones, implant stems are widely used as the primary design element for bone anchorage in a variety of implants. Concerning these implant stems, it is a common technique to anchor implant stems within the medullary cavity of a long bone using cement. An alternative technique is to use an uncemented implant stem that is first anchored during surgery by a press fit and is configured so that bone tissue can grow into the surface structure of the stem resulting in secondary fixation of the stem by bone ingrowth. However, the present invention relates to stems for cemented implantation.

In particular high-risk patients, for example patients suffering from diabetes, overweight and/or cancer, often require additional and instant fixation. Also, the phenomenon of aseptic loosening in these patients is more commonly observed. In such cases, even the technique of cemented implant stems frequently turns out not to be as reliable and as stable as desired.

FR 2 855 396 A1 discloses the subject-matter of the preamble of claim 1, namely a prosthesis including a free standing femoral rod having a sharp and sectional form with a set of openings inscribed in it, wherein the openings are filled with cement to place the rod in a diaphysis of a femur. The rod has lugs on its sides and connected to longitudinal terminal at the back of the rod.

WO 2019/171139 A1 relates to a temporary spacer device for a joint of the human body such as for example a knee joint comprising a first component or femoral component, adapted to be constrained to a bone or to the femoral bone of a patient, a second component or tibial component, adapted to be constrained to a bone or tibial bone of a patient, and at least one stem provided with a proximal end.

US 2016/235955 A1 discloses a temporary disposable spacer device implantable in the human body for treatment of an infected bone seat or a joint seat, which includes a body provided with at least one coupling surface to the bone seat or joint seat to be treated and recesses along the coupling surface.

DE 39 03 438 A1 relates to a hip joint endoprosthesis comprising a shaft and a connecting member facing a pelvis part, wherein the shaft comprises in its region of extension facing the connecting member a central element which connects the shaft that can be constructed from a modular system to the connection member that can be constructed from the modular system.

US 2006/149246 A1 relates to a modular intercalary implant system for use in replacing a missing portion of the shaft of a long bone.

### SUMMARY OF THE INVENTION

Thus, the objective of the invention is to provide a stem for an implant that offers permanent fixation with respect to fast, reliable and long-lasting stability and that prevents aseptic loosening.

The present disclosure relates to a stem for an implant (and to an implant comprising such a stem), which is adapted for cemented implantation, at least partially, into (parts of) the medullary cavity and/or a cavity created by surgery of a long bone, preferably a human humerus. The stem may also be adapted for bone-ingrowth.

The stem has a longitudinal axis and comprises at least one recess. Preferably, the recess is formed as a groove and even more preferably as a longitudinal groove, i. e. a groove that extends in the direction of or along the longitudinal axis of the stem. Nonetheless, the recesses may also be formed as holes or have any other shape that allows for incorporating cement.

Preferably, the stem comprises more than one recess, in particular two, three, four, five, or six recesses. The multiple recesses are circumferentially spaced or distributed about the longitudinal axis of the stem. Preferably, they are arranged parallel to each other.

According to the present disclosure, the recess, preferably each recess, is interrupted by at least one barrier. The barrier is oriented transverse to the longitudinal axis of the stem and protrudes from the bottom of the recess radially outwards. As a result, the barrier subdivides the recess into subrecesses along the stem. The barrier is preferably solidly integrated so as to be a part of the stem's shape and material, i. e. the barrier or protrusion forms a part of the stem.

This allows advantageously that cement on both sides of the barrier or protrusion supports fixation in both longitudinal directions of the stem and in the long bone and in particular in the medullary canal. More specifically, the barrier provides the means for establishing a form fit between the barrier and the bone cement in the recess on both sides of the barrier as well as between the bone tissue surrounding the implantation site, in particular cancellous bone tissue, and the implant stem by means of the bone cement. If having an elongated shape, the recess provides said form-fit and enhanced anchorage within the bone and at the same time facilitates removal during revision surgery.

For example, a stem as configured according to one of the embodiments of this disclosure can advantageously be used for anchoring a diaphyseal segment prosthesis, for example after a resection in cancer treatment. In case of a diaphyseal segment prosthesis, permanent fixation is advantageous since a need for replacement of the prosthesis is unlikely compared to other prostheses such as artificial joints. The latter particularly suffer from friction and wear and for this reason may require replacement during the lifetime of the patient.

Preferably, the recess has a minimum width of 1 mm, 2 mm, 3 mm, 4 mm, or 5 mm and a maximum width of 6 mm, 7 mm, 8 mm, or 10 mm. Further, the recess preferably has a depth of up to 0.5 mm, 1 mm, 2 mm, 3 mm, or 4 mm. If the recess has an elongated shape, it preferably has a length of at least 2 cm. Further, the recess preferably does not open up at the proximal or distal end of the stem.

These preferred properties of the recess enhance above-noted form-fit resulting from implantation. Generally, the bigger the recess the better the form fit. However, the biggest gains in anchoring strength are in the lower ranges of above-noted values.

In particular for being adapted for cemented implantation into the medullary cavity of a long bone, the outer contour of the cross section of the stem at the barrier is preferably continuous. In particular, at least one segment of the outer contour of the cross section of the stem at the barrier is round and in particular circular in order to allow smooth insertion into a cavity prepared in a long bone.

For the same reason, the outer surface of the barrier is cylindrical (and, in particular, has a round or circular cross-section) and/or forms a continuous part of an outer surface of the stem outside the recess, which is cylindrical as well (and, in particular, has a round or circular cross-section).

Further, the whole outer surface of the stem except for the at least one recess is cylindrical (and, in particular, has a circular cross-section).

This results in forming a continuous outer surface with a fully round or circular cylindrical outer surface of the barrier. Such a configuration allows on the one hand a smooth insertion of the stem during the surgical procedure and on the other hand facilitates the preparation of the cavity in the long bone for said insertion.

The stem may further be an element of a system of different elements, which are adapted to be mounted to each other in a variety of combinations to respectively form different implants. The stem may also be part of an implant and as such be integrally formed with the shape and material of the implant. For example, the stem may provide anchorage for an artificial joint, wherein the stem is configured to be implanted into a long bone. In particular, the stem may be configured for implantation into the humerus and may form a part of an artificial shoulder joint. In another example, two of the stems may provide anchorage for a diaphyseal segment prosthesis. Here, the diaphyseal segment prosthesis replaces a resected segment of a long bone, wherein the stems are adapted to be implanted into the resected ends of the remaining long bone. The skilled person will appreciate that this may, for example, apply to a humerus or a femur of a long bone.

### SHORT DESCRIPTION OF THE FIGURES

The following figures illustrate preferred embodiments of the present invention. These embodiments are not to be construed as limiting but merely to enhance the understanding of the invention together with the following description. The present invention is solely defined by the appended claims. In these figures, same reference signs refer to features throughout the drawings that have the same or an equivalent function and/or structure. In summary, the figures illustrate the following:
- Figure 1 shows a side view of an implant comprising two stems according to the disclosure;
- Figure 2 shows a perspective view of the implant of Figure 1 implanted into a human humerus;
- Figure 3 shows an exploded perspective view of the implant of Figure 1 ;
- Figure 4 shows a perspective view of an alternative implant assembled using one of the stems of the implant of Figure 1 ; and
- Figure 5 shows a perspective view of another alternative implant assembled using one of the stems of the implant of Figure 1 .

### DETAILED DESCRIPTION OF PREFERRED EMBODIMENTS

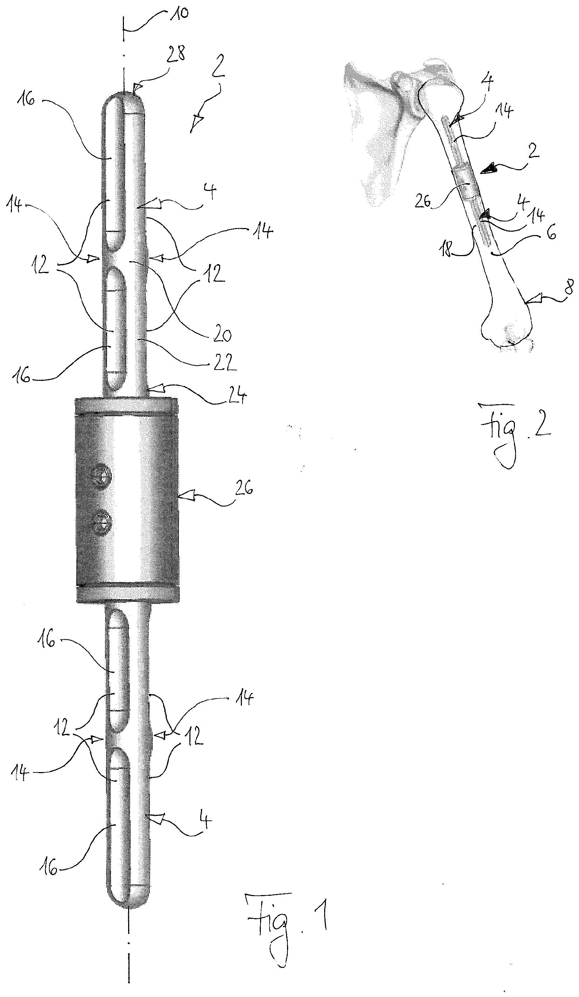

Figures 1 to 3 show an implant 2 (namely a diaphyseal segment prosthesis 2) comprising two stems 4 being adapted for cemented implantation into the medullary cavity 6 (after preparing the cavity 6 by surgery) of a long bone and in particular a human humerus 8. The implant 2 has a longitudinal axis 10. Each of the stems 4 in this exemplary embodiment comprises two recesses 12 in a circumferential direction of the stems 4. Nonetheless any number of recesses 12 in the circumferential direction of above may be formed. The same applies to above-noted dimensions and shaped for the recesses 12. In this exemplary embodiment, the recesses are formed as grooves, i. e. elongated recesses. As illustrated, they may be located parallel to each other, be on opposite sides of the stem and/or extend in the direction of or along the longitudinal axis 10.

Each recess 12 is interrupted by a barrier 14 in the longitudinal direction of the stem 4, which is preferably solidly integrated into the shape and material of the respective stem 4 and in particular about half way along the length of the respective stem. It is also possible to provide more than one barrier along a recess 12, such as two, three, four, or five barriers. Nonetheless, only one or two barriers is preferred.

The at least one barrier allows advantageously that cement 16 on both sides of the barrier 14 supports fixation in both longitudinal directions (against pulling and pushing forces in the humerus 8) by establishing a form fit. This also applies to bone tissue growing into the recesses 12 in case of cementless fixation using a stem 4 that has an outer surface adapted for bone ingrowth.

In case of fixation by bone cement, the form fit is established between the barrier 14 and the cement 16 in the recesses 12 on both sides of the barrier 14 as well as between bone tissue surrounding the implantation site of the stems 4, preferably cancellous bone tissue 18, and the respective stem. In case of cancellous bone tissue, the form fit may be enhanced by causing bone cement to enter into the cancellous bone's surface 18 resulting in a better interlock between the stem and the bone tissue. In case of fixation by bone ingrowth, a form fit is achieved directly between the bone tissue and the stem 4.

In particular for anchoring of a diaphyseal segment prosthesis 2 (e. g. after a resection in cancer treatment), permanent fixation, in particular using bone cement, is advantageous since a replacement of the prosthesis is unlikely compared to artificial joints. In comparison to a diaphyseal segment prosthesis, artificial joints are exposed to friction and wear during everyday use and may thus need replacement during the lifetime of the patient.

If being adapted for cemented implantation into the medullary cavity of a long bone, the outer contour of the cross section of the stem 4 at the barrier 14 is preferably continuous. It preferably has a smooth and even more preferably a polished surface. Further, the outer contour of the stem in cross-section along and except for the recesses 12 and barrier 14 is in particular round or circular (preferably forming a full circle). Likewise, the outer surface 20 of the barrier 14 is in particular round or circular (preferably forming a full circle). As described above, the outer contour of the stem 4 and barrier 14 is cylindrical.

The outer surface 20 of the barriers 14 preferably forms a continuous part of the outer surface 22 of the stem 4 outside its recesses 12. The outer surface 22 may be, as shown in the exemplary embodiment of the figures, circular cylindrical. In other words, the entire outer surface 22 of each stem 4 outside the recesses 12 and along the recesses is preferably circular cylindrical forming one continuous outer surface with an integrally formed and circular cylindrical outer surface 20 of the barriers 14. Compared to a tapered shape, the cylindrical shape has no influence on the depth of the recesses 12.

Each stem 4 comprises one connective end 24 adapted to be mounted to a diaphyseal segment body 26 and one continuously integrated preferably rounded and in particular semi-spherical distal end 28. The latter prevents damage to bone tissue during insertion.

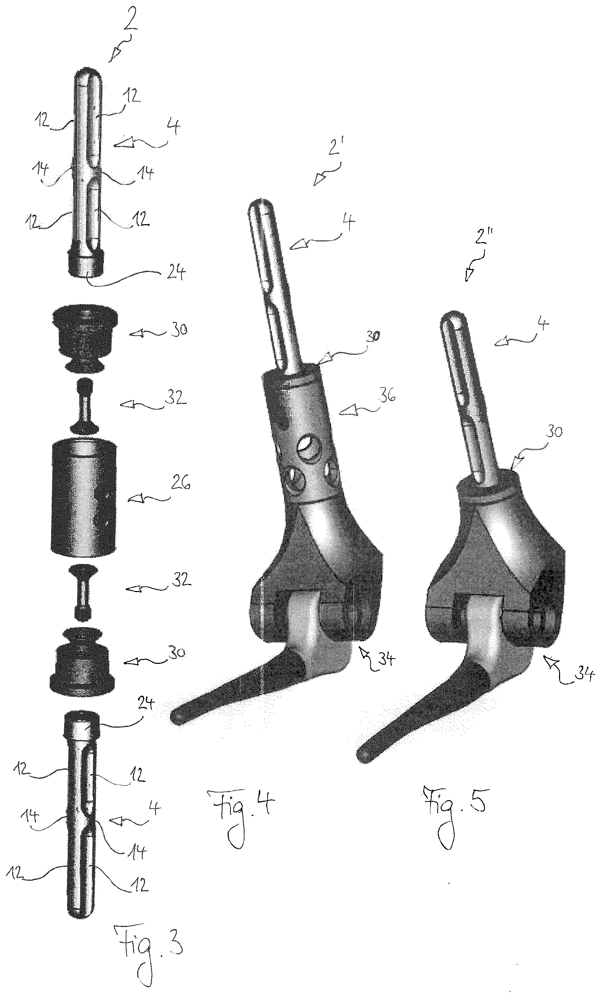

As shown in the exemplary illustrative embodiments of the figures, the stems 4 are members of a system of different elements like the diaphyseal segment body 26, the adapter 30, or the connective screw 32 (cf. figure 3 ). These elements are adapted for being mounted to each other in a variety of combinations to respectively form different implants 2, 2', 2".

For example, Figures 4 and 5 show implants 2' and 2", respectively, wherein the stem 4 is mounted to provide anchorage for an artificial elbow joint. The implants 2' and 2" are mounted as a combination of a variety of elements like the stem 4, adapter 30, and connective screw 32 also shown in Figure 3 , and additionally an elbow joint replacement 34 and a segment 36 for replacing a section of a long bone.

### REFERENCE SIGNS

- 2, 2', 2"
- implant
- 4
- stem
- 6
- medullary cavity
- 8
- human humerus
- 10
- longitudinal axis
- 12
- recess
- 14
- barrier
- 16
- cement
- 18
- cancellous bone
- 20
- outer surface of barrier 14
- 22
- outer surface of stem 4 outside the recess 12
- 24
- connective end
- 26
- diaphyseal segment body
- 28
- distal end
- 30
- adapter
- 32
- connective screw
- 34
- shoulder joint group
- 36
- joint adjacent segment

## Claims

1. Stem (4) for or of an implant (2), the stem being adapted for implantation into a cavity (6) of a long bone (8), wherein the stem has a longitudinal axis (10) and at least one recess (12), the at least one recess is interrupted by at least one barrier (14), wherein the barrier (14) is oriented transverse to the longitudinal axis (10), **characterized in that** a whole outer surface (22) of the stem (4) outside the recess (12) is cylindrical, and **in that** an outer surface (20) of the barrier (14) is cylindrical outside the recess (12).

2. Stem (4) according to claim 1, wherein the recess (12) is formed as a groove, preferably a longitudinal groove.

3. Stem (4) according to claim 1 or 2, wherein the barrier (14) is solidly integrated into the shape and material of the stem (4).

4. Stem (4) according to any one of the preceding claims, wherein the recess (12) has a minimum width of 1 mm, 2 mm, 3 mm, 4 mm, or 5 mm and a maximum width of 6 mm, 7 mm, 8 mm, or 10 mm.

5. Stem (4) according to any one of the preceding claims, wherein the recess (12) has a depth of up to 0.5 mm, 1 mm, 2 mm, 3 mm, or 4 mm.

6. Stem (4) according to any one of the preceding claims, wherein the outer contour (20) of the stem (4) at the barrier (14) is continuous.

7. Stem (4) according to any one of the preceding claims, wherein the outer contour (20) of the stem (4) at the barrier (14) is circular.

8. Stem (4) according to any one of the preceding claims, wherein at least a segment of the outer surface (20) of the barrier (14) forms a continuous part of an outer surface (22) of the stem (4) outside the recess (12).

9. Stem (4) according to anyone of the preceding claim, wherein the geometry of the stem (4) is adapted to be at least partly implanted into the medullary cavity (6) of a humerus (8).

10. Stem (4) according to any one of the preceding claims, wherein the stem (4) is an element of a system of different elements (4, 26) adapted to be mounted together in a variety of combinations to form respective different implants.

11. Stem (4) according to any one of the preceding claims, wherein the at least one barrier (14) is a single barrier (14) or two barriers (14).

12. Implant (2) comprising a stem (4) according to any one of the preceding claims.

## Patentansprüche

1. Schaft (4) für ein oder von einem Implantat (2), wobei der Schaft zur Implantation in einen Hohlraum (6) eines langen Knochens (8) angepasst ist, wobei der Schaft eine Längsachse (10) und mindestens eine Vertiefung (12) aufweist, wobei die mindestens eine Vertiefung durch mindestens eine Barriere (14) unterbrochen ist,
wobei die Barriere (14) quer zu der Längsachse (10) ausgerichtet ist, **dadurch gekennzeichnet, dass** eine ganze Außenfläche (22) des Schafts (4) außerhalb der Vertiefung (12) zylindrisch ist und dadurch, dass eine Außenfläche (20) der Barriere (14) außerhalb der Vertiefung (12) zylindrisch ist.

2. Schaft (4) nach Anspruch 1, wobei die Vertiefung (12) als eine Nut, vorzugsweise eine Längsnut, ausgebildet ist.

3. Schaft (4) nach Anspruch 1 oder 2, wobei die Barriere (14) fest in die Form und das Material des Schafts (4) integriert ist.

4. Schaft (4) nach einem der vorhergehenden Ansprüche, wobei die Vertiefung (12) eine Mindestbreite von 1 mm, 2 mm, 3 mm, 4 mm oder 5 mm und eine Höchstbreite von 6 mm, 7 mm, 8 mm oder 10 mm aufweist.

5. Schaft (4) nach einem der vorhergehenden Ansprüche, wobei die Vertiefung (12) eine Tiefe von bis zu 0,5 mm, 1 mm, 2 mm, 3 mm oder 4 mm aufweist.

6. Schaft (4) nach einem der vorhergehenden Ansprüche, wobei die Außenkontur (20) des Schafts (4) an der Barriere (14) fortlaufend ist.

7. Schaft (4) nach einem der vorhergehenden Ansprüche, wobei die Außenkontur (20) des Schafts (4) an der Barriere (14) kreisförmig ist.

8. Schaft (4) nach einem der vorhergehenden Ansprüche, wobei mindestens ein Abschnitt der Außenfläche (20) der Barriere (14) einen fortlaufenden Teil einer Außenfläche (22) des Schafts (4) außerhalb der Vertiefung (12) ausbildet.

9. Schaft (4) nach einem der vorhergehenden Ansprüche, wobei die Geometrie des Schafts (4) zum mindestens Teilimplantieren in die Markhöhle (6) eines Oberarmknochens (8) angepasst ist.

10. Schaft (4) nach einem der vorhergehenden Ansprüche, wobei der Schaft (4) ein Element eines Systems verschiedener Elemente (4, 26) ist, die zum Zusammenmontieren in einer Vielfalt von Kombinationen unter Ausbilden jeweiliger unterschiedlicher Implantate angepasst sind.

11. Schaft (4) nach einem der vorhergehenden Ansprüche, wobei die mindestens eine Barriere (14) eine einzelne Barriere (14) oder zwei Barrieren (14) ist.

12. Implantat (2), umfassend einen Schaft (4) nach einem der vorhergehenden Ansprüche.

## Revendications

1. Tige (4) pour ou d'un implant (2), la tige étant conçue pour une implantation dans une cavité (6) d'un os long (8), dans laquelle la tige a un axe longitudinal (10) et au moins un évidement (12), l'au moins un évidement est interrompu par au moins une barrière (14),
dans laquelle la barrière (14) est orientée transversalement à l'axe longitudinal (10), **caractérisée en ce qu'**une surface externe (22) de la tige (4) à l'extérieur de l'évidement (12) est cylindrique, et **en ce qu'**une surface externe (20) de la barrière (14) est cylindrique à l'extérieur de l'évidement (12).

2. Tige (4) selon la revendication 1, dans laquelle l'évidement (12) est formé comme une rainure, de préférence une rainure longitudinale.

3. Tige (4) selon la revendication 1 ou 2, dans laquelle la barrière (14) est solidement intégrée à la forme et au matériau de la tige (4).

4. Tige (4) selon l'une quelconque des revendications précédentes, dans laquelle l'évidement (12) a une largeur minimale de 1 mm, 2 mm, 3 mm, 4 mm ou 5 mm et une largeur maximale de 6 mm, 7 mm, 8 mm ou 10 mm.

5. Tige (4) selon l'une quelconque des revendications précédentes, dans laquelle l'évidement (12) a une profondeur allant jusqu'à 0,5 mm, 1 mm, 2 mm, 3 mm ou 4 mm.

6. Tige (4) selon l'une quelconque des revendications précédentes, dans laquelle le contour externe (20) de la tige (4) au niveau de la barrière (14) est continu.

7. Tige (4) selon l'une quelconque des revendications précédentes, dans laquelle le contour externe (20) de la tige (4) au niveau de la barrière (14) est circulaire.

8. Tige (4) selon l'une quelconque des revendications précédentes, dans laquelle au moins un segment de la surface externe (20) de la barrière (14) forme une partie continue d'une surface externe (22) de la tige (4) à l'extérieur de l'évidement (12).

9. Tige (4) selon une quelconque revendication précédente, dans laquelle la géométrie de la tige (4) est conçue pour être au moins partiellement implantée dans la cavité médullaire (6) d'un humérus (8).

10. Tige (4) selon l'une quelconque des revendications précédentes, dans laquelle la tige (4) est un élément d'un système de différents éléments (4, 26) conçus pour être montés ensemble dans une variété de combinaisons afin de former des implants différents respectifs.

11. Tige (4) selon l'une quelconque des revendications précédentes, dans laquelle l'au moins une barrière (14) est une barrière unique (14) ou deux barrières (14).

12. Implant (2) comprenant une tige (4) selon l'une quelconque des revendications précédentes.
